# EUROPEAN PATENT SPECIFICATION

(11) **EP 1 924 310 B1**
(45) Date of publication and mention of the grant of the patent: **20.04.2016**
(21) Application number: 06813621.7
(22) Date of filing: 21.08.2006
(51) Int. Cl.: A61M 16/08, A61M 15/00, A61M 16/20

(54) **Self-sealing T-piece and valved T-piece**
Selbstversiegelndes T-Stück und Ventil-T-Stück
Pièce en T auto-étanche et pièce en T pourvue d'une valve

(30) Priority: 23.08.2005 US 710932 P
(43) Date of publication of application: 28.05.2008
(73) Proprietor: Nektar Therapeutics, San Francisco, CA 94158 (US)
(72) Inventor: FINK, James B., San Mateo, CA 94402 (US); O'SULLIVAN, Gavan, COUNTY GALWAY (IE); DUNNE, Paul, Dublin 9 (IE); MORAN, Declan, County Galway (IE); POWER, John, County Galway (IE); SMITH, Niall, Galway (IE)
(74) Representative: Boult Wade Tennant
(86) International application number: PCT/US2006/032677
(87) International publication number: WO 2007/024812

(56) References cited:
- WO-A-01/83011
- US-A- 4 930 498
- US-A- 4 938 210
- US-A- 5 396 883
- US-A- 6 014 972
- US-A1- 2003 150 445

## Description

### BACKGROUND OF THE INVENTION

This invention relates to apparatus and components for coupling fluid or gas conducting elements, such as apparatus and components for connecting a nebulizer (also known as an aerosol generator) with a gas flow system. In particular, the present invention relates to apparatus and components for connecting a nebulizer to a pressure-assisted breathing system, such as a mechanical ventilator or a continuous positive airway pressure ("CPAP") system. As used herein, the term "pressure-assisted breathing system" means any artificial ventilation system that applies continuous or intermittent pressure, usually positive, (i.e. above a certain baseline such as atmospheric pressure), to gas(es) in or about a patient's airway during inhalation as a means of augmenting movement of gas(es) into the lungs. The present invention is contemplated as being useful in any pressure-assisted breathing system and "pressure-assisted breathing system" is intended to include, for example, standard CPAP, nCPAP and Bi-level CPAP systems as well as mechanical ventilators that perform the breathing function for the patient and/or provide CPAP to assist in spontaneous breathing by the patient. The term "pressure-assisted breathing system" is also intended to include both invasive and non-invasive systems. Systems that utilize an endotracheal or tracheostomy tube are examples of invasive pressure-assisted breathing systems. Systems that utilize nasal prongs or a mask are examples of non-invasive pressure-assisted breathing systems.

Pressure-assisted breathing systems utilize positive pressure during inhalation to increase and/or maintain lung volumes and to decrease the work of breathing by a patient. The positive pressure effectively dilates the airway and prevents its collapse. The delivery of positive airway pressure may be accomplished through the use of a positive air flow source ("flow generator") that provides oxygen or a gas containing oxygen through a flexible tube connected to a patient interface device such as nasal prongs (cannula), nasopharyngeal tubes or prongs, an endotracheal tube, mask, etc. The tubes associated with commercially available pressure-assisted breathing systems create a "circuit" for gas flow by maintaining fluid communication between the elements of the circuit. Tubes may be made of a variety of materials, including but not limited to various plastics, metals and composites and can be rigid or flexible.

A nebulizer may be connected to a circuit of a pressure-assisted breathing system to deliver an aerosol of medication (sometimes herein referred to as "aerosolized medicament") into the respiratory system of a patient. The nebulizer is adapted to emit aerosolized medicament into the gas flow in the circuit, which delivers the aerosol to the patient through a patient interface device. Nebulizers suitable for the practice of the present invention preferably comprise a reservoir for holding a liquid medicament to be delivered to a patient's respiratory system and a vibrating aperture-type aerosol generator for aerosolizing the liquid medicament. The nebulizer is typically connected to the circuit using a generally "T"-shaped connector (sometimes referred to herein as a "T"-piece). For example, U.S. Pat. Nos. 6,615,824; 6,948,491; 6,615,824; 7,100,600; 6,968,840; 7,322,349; 7,290,541; 7,267 and US Publication Nos. 2002-0134375; 2002-013434; 2003-0150445; 2005-0229927 and 2005-0217666 describe apparatuses and methods for ventilators and connecting nebulizers to pressure-assisted breathing systems.

Pressure-assisted breathing systems such as those described in the patents and pending applications cited above must have leak-free, or gas-tight, circuits to maintain adequate pressure. As a result, ventilation and positive airway pressure may have to be interrupted in those systems to insert and withdraw the nebulizer from the circuit, for example, when the nebulizer needs to be refilled with liquid medicine, or needs cleaning, replacement, adjustment or repair. Furthermore, it is often desirable to prevent or mitigate escape of gases and/or aerosolized materials, for example, medicaments, from within the circuit. Additionally it is desirable that replacement of a nebulizer in a gas circuit be accomplished quickly, and with a minimum of effort.

WO 01/83011 discloses a spacer assembly for a ventilator or other respiratory equipment for dispensing aerosol drugs from a metered dose inhaler, canister or nebulized drugs from a nebulizer port into a respiratory gas stream delivered from a ventilator or other respiratory equipment connected to a patient. The ventilator spacer body includes a first portion and a second portion. The second portion preferably includes an aperture for cooperating with a valve assembly which is preferably constructed as a gate valve and includes a base adhered to the inside face of the second body portion and a cylindrical throat extending from the base and having an opening for receiving a discharge outlet of a conventional nebulizer. When disconnected from the nebulizer the valve seals the opening using a gate biased to a close position by a torsion spring. When the nebulizer discharge outlet is inserted through the aperture it urges the gate from the seating contact to an open position. When the nebulizer discharge outlet is withdrawn the gate returns to its seated position.

### SUMMARY OF THE INVENTION

According to the present invention there is provided an apparatus for delivery of medicament to a breathing system as claimed in the appended claims.

In particular, the invention relates to apparatus comprising a nebulizer that is adapted to aerosolize a liquid medicament and a connector that operably connects the nebulizer to a circuit of the pressure-assisted breathing system.

In one or more embodiments a connector of the present invention comprises a gas conduit, an aerosol supply conduit and a sealing device configured to seal the aerosol supply conduit from the gas conduit when a nebulizer is not connected and provide an unimpeded path for aerosol when the nebulizer is connected. The gas conduit has an inlet opening and an outlet opening adapted to be attached to a circuit of the pressure-assisted breathing system so that the flow of gas in the circuit is conducted therethrough. The aerosol supply conduit has an inlet opening adapted to receive the nebulizer and an outlet opening that communicates with the gas conduit so that aerosolized medicament produced by the nebulizer passes through the aerosol conduit and into the gas flow in the gas conduit. The sealing device is configured to allow unimpeded flow of aerosolized medicament through the aerosol supply conduit into the gas conduit when the nebulizer is positioned in the aerosol supply conduit, and to seal off the aerosol supply conduit from the gas conduit when the nebulizer is removed therefrom.

The sealing device comprises a hinged door with a spring-loaded or biased hinge positioned on the interior wall of the gas conduit adjacent to the outlet opening of the aerosol supply conduit. When inserted into the inlet opening of the aerosol supply conduit, the nebulizer forces the hinged door into a notch or recess in the internal surface of the gas conduit, thereby providing a first position in which unimpeded flow of aerosol from the nebulizer into the gas conduit is provided. When the nebulizer is removed, the spring on the hinge forces the door to a close over the outlet opening of the aerosol supply conduit to seal off the aerosol supply conduit from the gas conduit.

In one or more embodiments, the coupling device or assembly provides a gas-tight seal between components.

In one or more embodiments, the coupling device or assembly provides a quick-release connection between components.

In one or more embodiments, the coupling device or assembly provides both a gas-tight seal, and a quick-release connection between components.

### BRIEF DESCRIPTION OF THE DRAWINGS

FIG. 1a is a perspective view of a known apparatus for the delivery of a medicament to a pressure-assisted breathing system.
FIG 1b is an enlarged, cross-section view of a known connector similar to the one shown in FIG. 1a, wherein a commercially available spring-loaded valve is used to prevent release of gas flowing in a pressure-assisted breathing system when a nebulizer is not inserted in the connector.
FIG. 1c is an enlarged, cross-section view of a known apparatus using the connector shown in FIG. 1b, wherein the spring-loaded valve is actuated by insertion of a nebulizer into the-connector to allow aerosol to enter the gas flowing through the connector in a pressure-assisted breathing system.
FIG. 2 is a perspective view of one arrangement of a connector, wherein a spring-loaded or biased lid is disposed over an inlet opening of an aerosol supply conduit.
FIG. 3 is an enlarged, cross-section view of the connector of FIG. 2 showing movement of the lid.
FIG. 4a is a prospective view of a flap seal for positioning in the connector shown in FIG. 3.
FIG. 4b is a cross-section view of the flap seal shown in FIG. 4a wherein the flaps of the seal are deflected by the barrel of a nebulizer inserted therein.
FIG. 5 is an exploded, perspective view of another arrangement of apparatus , wherein a rotatable disc adapter with an off-set opening is positioned between the nebulizer and an off-set inlet opening of an aerosol supply conduit.
FIG. 6a is an exploded, top-view of the connector and adapter shown in FIG. 5 when in the closed position that seals off an aerosol supply conduit from a gas conduit.
FIG. 6b is an exploded, top-view of the connector and adapter shown in FIG 5 when in the open position that provides an unimpeded path for aerosol from the nebulizer to a gas conduit.
FIG. 7a is an enlarged, cross-section view of one embodiment of a connector according to the present invention in closed position wherein a spring-loaded or biased hinged door covers an outlet opening of an aerosol supply conduit to seal off the aerosol supply conduit from a gas conduit.
FIG. 7b is an enlarged, cross-section view of apparatus using the connector of FIG. 7a in open position wherein the spring-loaded or biased hinged door is deflected into a notch or recess in an internal surface of a gas conduit to provide an unimpeded path for aerosol from the nebulizer to the gas conduit.
FIG. 8a is a perspective view of another arrangement of a connector wherein an aerosol supply conduit comprises a rotatable sleeve through which a gas conduit is placed.
FIG. 8b is an exploded, perspective view of the connector shown in FIG. 8b.

### DESCRIPTION OF THE INVENTION

Unless otherwise stated, a reference to a compound or component includes the compound or component by itself, as well as in combination with other compounds or components, such as mixtures of compounds.

As used herein, the singular forms "a," "an," and "the" include the plural reference unless the context clearly dictates otherwise.

Medicament, "active agent" or pharmaceutical may be used interchangeably, and individually or collectively comprise any drug, solution, compound or composition which induces a desired pharmacologic and/or physiologic effect, when administered appropriately to the target organism (human or animal).

Reference herein to "one embodiment", "one version" or "one aspect" shall include one or more such embodiments, versions or aspects, unless otherwise clear from the context.

As an overview, the present invention comprises apparatus, systems, assemblies, components and ventilator circuits. In some embodiments, one or more components may be used independently of the other combinations and/or assemblies described herein. Moreover, the various embodiments of the coupling apparatus are not limited to use with the ventilator circuits of the invention. Thus the various embodiments of the coupling apparatus of the present invention may be used in a variety of fluid and/or gas flow applications where a device to atomize a fluid is to be incorporated into a fluid or gas supply system. This includes, without limitation, systems for distributing or supplying an aerosolized material within a gas or fluid manifold or distribution circuit, such as fuel supply systems, coating systems, biological test systems and the like. Such systems can have aesthetic purposes, as for example, distributing a fragrance or other aesthetic component, or may be for functional purposes.

One or more embodiments of the apparatus, systems, assemblies and components are configurable to administer aerosolized medicament to a patient on-ventilator or off-ventilator. On-ventilator treatment methods comprise administering the nebulized aerosol through a ventilator circuit to the patient. Aerosol doses, containing an effective dose, such as about 1 to about 500 mg of a medicament, may be delivered through the ventilator circuit in a phasic or non-phasic manner. Off-ventilator treatment methods comprise taking the patient off the ventilator before administering the nebulized aerosol. Once the treatment session is completed the patient may be put back on the ventilator, or may breathe on his or her own without assistance. Off-Vent devices often are self-contained, for freely-breathing patients, and may comprise an aerosol generator (e.g. a nebulizer) and a mask, cannula, lipseal or mouthpiece to administer an aerosolized liquid or powder formulation, such as a medicament. Administration may be continuous, phasic (such as during inspiration), and/or intermittent (such as timed). Devices, especially off-vent devices, used to administer the aerosol formulations, such as medicaments, may comprise a reservoir or holding chamber to permit or allow continuous flow of aerosol. While one benefit of the apparatus, systems, assemblies, components of the present invention is in conjunction with positive pressure-type apparatus, the apparatus, systems assemblies and components of the present invention may also be useful in non-pressurized systems, neutral pressure systems, or negative pressure (e.g. vacuum) systems, as being rapidly and easily replaceable, exchangeable or interchangeable.

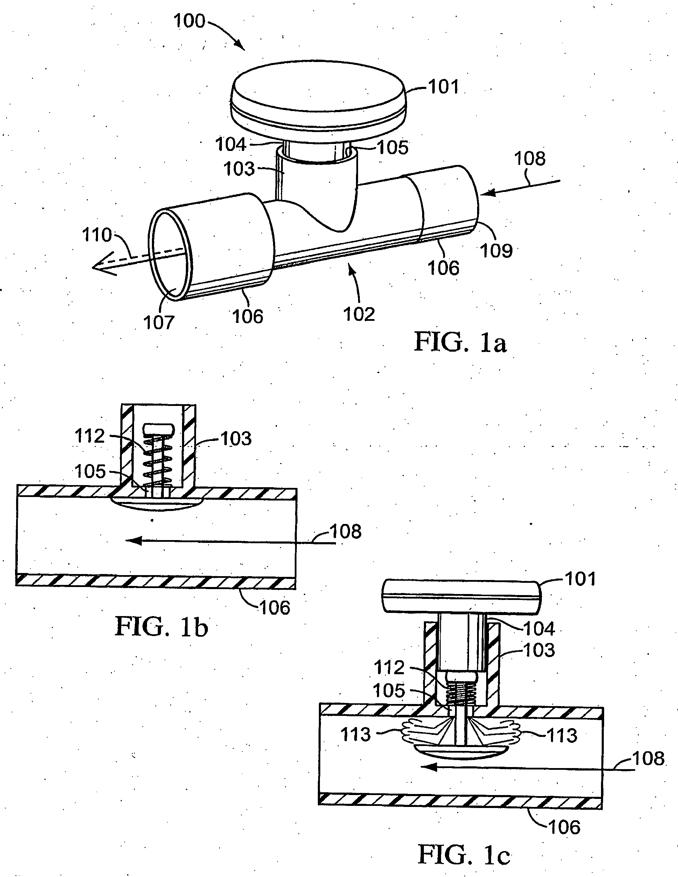

Referring to the drawings and initially to FIG. 1a thereof, there is illustrated a known aerosolized medicament delivery apparatus 100 that is suitable for coupling with a circuit of a pressure-assisted breathing system connected to the respiratory system of a patient, for example as described in detail in the aforementioned U.S. Pat. No. 6,615,824.In its most basic form, delivery apparatus 100 comprises a nebulizer 101 having an aerosol generator (not shown) for aerosolizing a liquid medicament, and a generally "T"-shaped hollow connector 102 for coupling nebulizer 101 to a circuit of a pressure-assisted breathing system. Although reference is made herein for convenience to a "T"-shaped connector, or "T"-piece, it is understood that the connector 102 may have other shapes, for example, a "Y" or other shape.

Connector 102 comprises an aerosol supply conduit 103 having inlet opening 105 into which barrel 104 of nebulizer 101 may be inserted, a gas conduit 106 having an inlet opening 109, which may be attached to one tube of the pressure-assisted breathing system circuit, and outlet opening 107, which may be attached to another tube of the circuit, thereby completing the circuit through gas conduit 106. Gas flow 108 flowing under positive pressure in the circuit enters inlet opening 109 and is conducted to the junction of aerosol supply conduit 103 and gas conduit 106. An aerosol of medication generated by nebulizer 101, preferably using a vibrating aperture-type aerosol generator, passes through barrel 104 into aerosol supply conduit 103 and into the junction, where it is entrained in gas flow 108 to form gas flow 110 comprising entrained aerosolized medicament. Gas flow 110 exits gas conduit 106 through outlet opening 107 into the pressure-assisted breathing system circuit. The aerosol of medicine is then ultimately carried by the gas flow in the pressure-assisted breathing system to the patient's respiratory system, e.g. through a patient interface device. When nebulizer 101 is withdrawn from connector 102 in the arrangement illustrated in Fig. 1a, gas flows 108 and 110 may be disrupted since they will be diverted into aerosol supply conduit 103 and out opening 105 to the atmosphere, thereby eliminating the positive airway pressure in the circuit.

FIG. 1b illustrates one proposal that has been advanced to solve the above-described problem. As shown in FIG. 1b, a spring-loaded valve 112 is positioned in aerosol supply conduit 103 of connector 102. The force of the spring holds valve 112 over inlet opening 105 when barrel 104 of nebulizer 101 is not present in aerosol supply conduit 103, and therefore valve 112 seals off aerosol inlet opening 105. As shown in FIG. 1c, when barrel 104 is inserted into aerosol supply conduit 103, valve 112 is forced downwards by barrel 104 and inlet opening 105 is uncovered, thereby allowing aerosol 113 emitted by nebulizer 101 to be released into and entrained by air flow 108. An example of a commercially available "T"-piece is provided by Thayer Medical and is designed to work with a pneumatic nebulizer. Although this device may effectively seal the circuit when nebulizer 101 is withdrawn, the efficiency of delivery of aerosol to the pressure-assisted breathing system is drastically reduced since valve 112 is in the path of the aerosol and impedes its flow. As shown in FIG. 1c, aerosol 113 impacts valve 112 and is deflected from gas flow 108 through gas conduit 106.

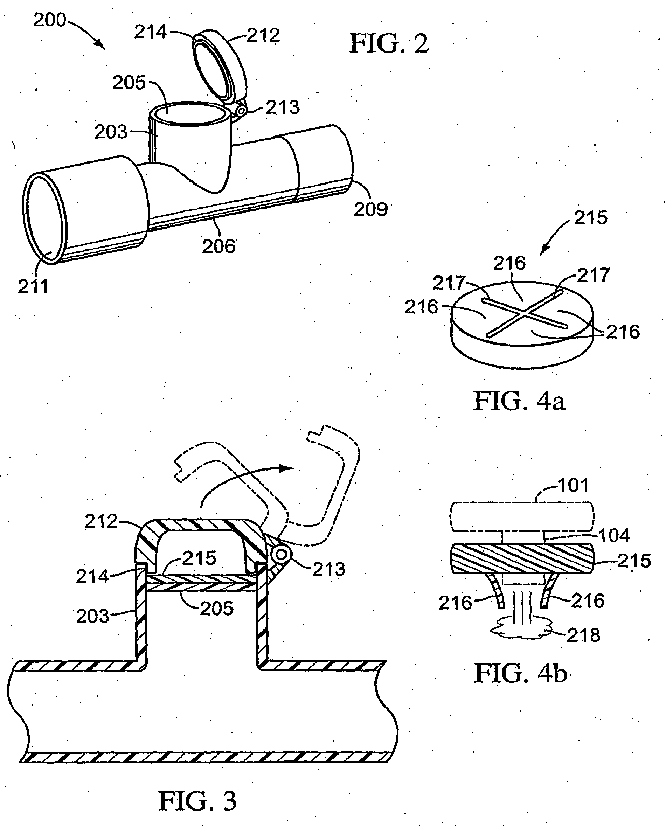

FIGS. 2, 3, 4a and 4b illustrate alternative arrangements of a connector wherein insertion and removal of a nebulizer from the aerosol supply conduit may be accomplished without interrupting the positive pressure gas flow in the circuit of the pressure-assisted breathing system with which it is coupled, while maintaining a high efficiency of aerosol delivery, and further without impeding flow of aerosol or gas. In one or more arrangements, there is provided a connector 200, which comprises aerosol supply conduit 203 having inlet opening 205 into which a nebulizer barrel may be inserted, gas conduit 206 having inlet opening 209 for the entrance of gas from a circuit, and outlet opening 211 for the exit of entrained aerosol and gas into the circuit. In addition, connector 200 has a hinged lid 212 attached to aerosol supply conduit 203 by spring-loaded or otherwise biased hinge 213. As shown in FIG. 3, lid 212 is held in a closed position over inlet opening 205 by the hinge 213 when the nebulizer barrel is not present, thereby sealing the circuit and maintaining positive pressure. Lid 212 may be rotated upwards to uncover inlet opening 205 and accommodate the insertion of the nebulizer barrel when desired. This position provides an unimpeded path for aerosol to travel from the nebulizer to the gas conduit. Upon removal of the nebulizer barrel, spring-loaded hinge 213 causes lid 212 to return to the closed position over inlet opening 205 to re-seal the circuit.

In some arrangements, lid 212 may have an "O"-ring seal 214 around its lower periphery to aid in the sealing of inlet opening 205. In some arrangements, a slotted flap seal or valve 215 may be positioned in inlet opening 205, as shown in FIG. 3. Slotted flap seal 215 is preferably made of a suitable sealing material, e.g. silicone, and may comprise a plurality of deformable flaps 216 defined by crossed slots 217, as illustrated in FIG. 4a. FIG. 4b illustrates the deflection of flaps 216 when nebulizer barrel 104 of nebulizer 101 is inserted into inlet opening 205. The deflection of flaps 216 allows unimpeded flow of aerosolized medicament 218 from nebulizer through aerosol conduit 203. When nebulizer barrel 104 is removed from inlet opening 205, flaps 216 return to their original position to re-seal the circuit before lid 212 is returned to the closed position shown in FIG. 3. Closed flap seal 215 also aids in maintaining the seal when the nebulizer is removed. In some arrangements, the slotted flap seal 215 may be made sufficiently robust to function alone to seal the inlet opening 205, yet permit insertion of the nebulizer barrel 104. In such arrangements, the lid 212 and hinge 213 may be omitted.

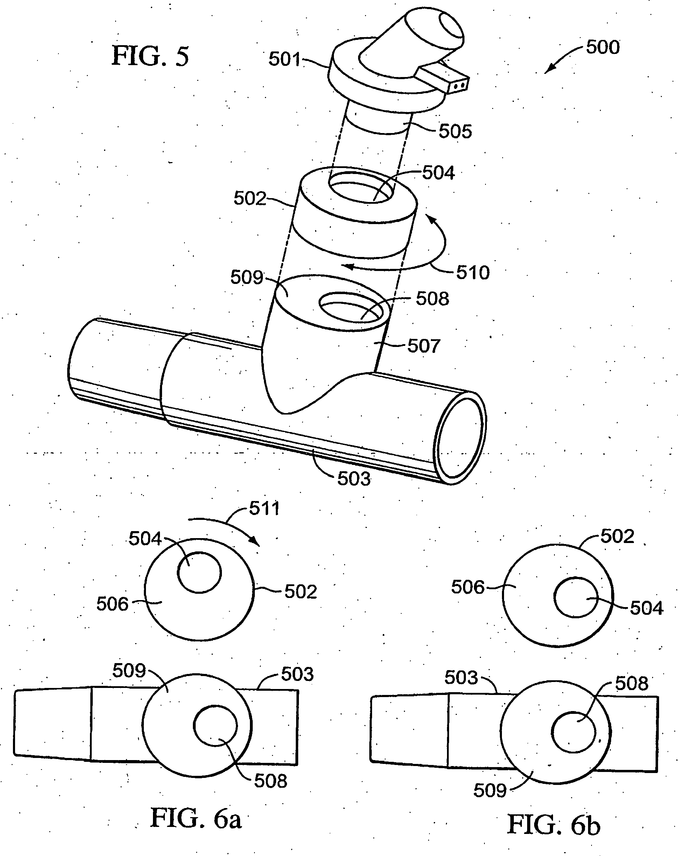

FIG. 5 illustrates another arrangement of the apparatus represented by the reference character 500. Apparatus, 500 comprises a nebulizer 501, a rotatable disc adaptor 502 and a sleeve connector 503. Adapter 502 has an off-set opening 504 in planar surface 506 into which barrel 505 of nebulizer 501 may be inserted. The offset opening 504 is preferably off-set about a central axis of the adapter 502. Sleeve connector 503 comprises aerosol supply conduit 507 having off-set inlet opening 508 in planar surface 509 that is substantially equal in size and shape to opening 504 in adaptor 502. The offset opening 508 is also preferably off-set about a central axis of the surface 509. Adaptor 502 is preferably mounted on connector 503 such that it can be rotated about a central axis as indicated by arrows 510. Accordingly, adapter 502 may be placed in the position shown in the overhead view of FIG. 6a during removal or insertion of nebulizer 501 into opening 504. In the position illustrated by FIG. 6a, opening 504 in adapter 502 is not aligned with opening 508 in aerosol supply conduit 507 and therefore opening 508 is effectively blocked by planar surface 506 of adaptor 502 (and opening 504 is effectively blocked by planar surface 509 of aerosol supply conduit 503). This position seals the circuit and allows barrel 505 of nebulizer 501 to be removed from opening 504 without interrupting gas flow through connector 503 or losing pressure in the pressure-assisted breathing system. When it is desired to connect nebulizer 501 to connector 503, barrel 505 is inserted in opening 504 and adapter 502 is rotated in the direction of arrow 511 to the position illustrated in FIG. 6b. In this position, opening 504 is aligned with opening 508, thereby providing an unimpeded path for aerosol emitted from nebulizer 501 to enter aerosol supply conduit 507 of connector 503 and become entrained in the gas flow of the system such as a pressure-assisted breathing system, as previously described. If required, it is contemplated that suitable sealing means (such as O-rings, gaskets or the like) may be placed between adapter 502 and planar surface 509 to further reduce the possibility of leaks. Additionally, suitable detents, indents, tabs or indicators (not shown) maybe provided on appropriate engaging surfaces of conduit 507 and adaptor 502 to positively identify engagement and disengagement of openings 504 and 508.

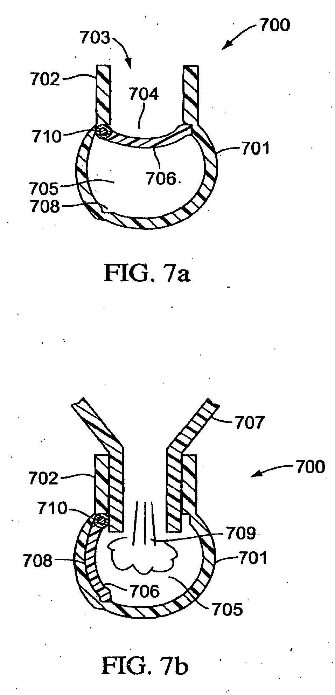

FIGS. 7a and 7b illustrate an embodiment of the invention designated by the general reference character 700, and represent a cross-section view perpendicular to the longitudinal axis of the connector 700. Connector 700 has an aerosol supply conduit 702 having an inlet opening 703 at its distal end and an outlet opening 704 at its proximal end. Outlet opening 704 communicates with gas conduit 705 running the length of the main body of connector 700. Hinged door 706 is attached to the internal wall of gas conduit 705 by spring-loaded or otherwise biased hinge 710. In the position illustrated in FIG 7a, door 706 is disposed over outlet opening 704 to seal aerosol supply conduit 702 and prevent the escape of the gas flow in gas conduit 705. When nebulizer barrel 707 is inserted into aerosol supply conduit 702 through inlet opening 703, nebulizer barrel 707 forces hinged door 706 downward, opening a pathway for the aerosol 709. There is provided a notch or recess 708 in the internal wall of gas conduit 705 to further facilitate insertion of the nebulizer barrel 707 and to provide an unimpeded path for aerosol 709 from the nebulizer into the gas flow in gas conduit 705, as shown in FIG. 7b. In an embodiment, the notch or recess 708 may cooperate with a mating structure (not shown) on the door 706 to assist in removably holding the door 706 in the open position, and/or aligning the door 706. When nebulizer barrel 707 is withdrawn from aerosol supply conduit 702, spring-loaded or biased hinge 710 acts to return hinged door 706 to the closed position shown in FIG. 7a, thereby re-sealing the circuit. The hinge or biasing means 710 may be configured to hold the door 706 between two positions, such as an open position as depicted in FIG 7b and a closed position as depicted in FIG 7a.

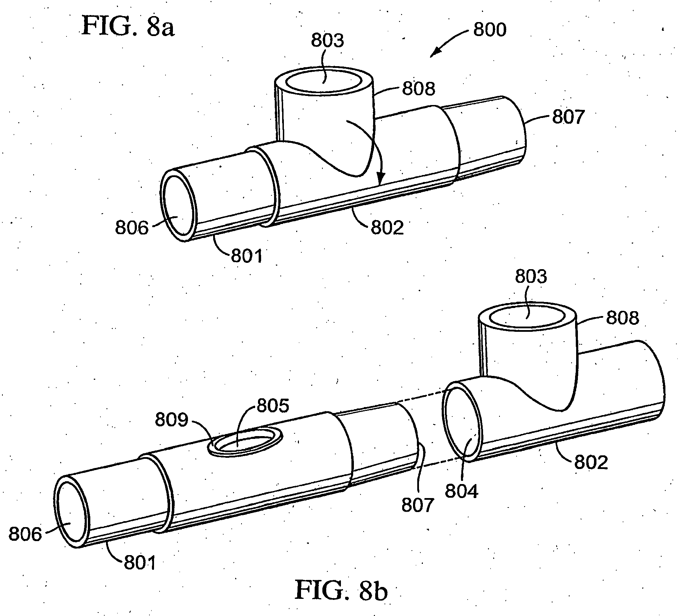

Another arrangement is illustrated in FIGS. 8a and 8b, wherein connector 800 comprises gas conduit 801 and rotatable sleeve 802, which may be rotatable about the conduit 801. Rotatable sleeve 802 comprises aerosol supply conduit 808 and lumen 804, which is configured to receive gas conduit 801 so that aerosol supply conduit 808 can rotate around the longitudinal axis of gas conduit 801. Aerosol supply conduit 808 comprises inlet opening 803 configured to accommodate the barrel of a nebulizer, as previously described. Gas conduit 801 comprises intermediate opening 805, outlet opening 806 and inlet opening 807. Gas conduit 801 may be positioned within lumen 804 of sleeve 802 so that intermediate opening 805 in gas conduit 801 is aligned with aerosol supply conduit 803, as shown in FIG. 8a. When a nebulizer is inserted in inlet opening 803, the position shown in FIG. 8a allows an unimpeded path for aerosol emitted by the nebulizer into aerosol supply conduit 808 to travel through intermediate opening 805 into gas conduit 801 and be entrained in the gas flowing from inlet opening 807 to outlet opening 806. When the nebulizer is to be removed from connector 800, sleeve 802 may be rotated relative to conduit 801 in the direction indicated by the arrow in FIG 8a to a position wherein aerosol supply conduit 803 is effectively blocked by the internal wall of lumen 804. In this position, the aerosol supply conduit 808 is sealed off from gas conduit 801 and the nebulizer may be removed from connector 800 without the escape of gas from gas conduit 801. When the nebulizer is re-inserted into aerosol supply conduit 808 through inlet opening 803, sleeve 802 may be rotated to the position shown in FIG. 8a, thereby again opening sup an unimpeded path for aerosol to pass through aerosol supply conduit 808 into the gas flow of gas conduit 808. Optionally a suitable sealing means 809, such as an O-ring, gasket or the like, may be placed around opening 805 to further reduce the possibility of leakage. Additionally, suitable detents, indents, tabs or indicators (not shown) may be provided on appropriate engaging surfaces of conduit 801 and sleeve 802 to positively identify engagement and disengagement of openings 803 and 805.

The aerosol generators or nebulizers contemplated for use herein may, for example, be a vibrating mesh nebulizer where the energy source is mechanical, such as wave energy, an ultrasonic nebulizer where the energy source is acoustic wave energy, a jet nebulizer where the energy source is compressed air, a metered dosing device where the energy source is a propellant, such as a composition that boils under preselected, such as ambient conditions, or a dry powder device where the energy source is compressed or flowing air or is a vibrating membrane or the like.

Some specific, non-limiting examples of technologies for producing fine liquid droplets comprise those which supply liquid to an aperture plate having a plurality of tapered apertures, and vibrate the aperture plate to eject liquid droplets through the apertures. Such techniques are described generally in U.S. Pat. Nos. 5,164,740; 5,938,117; 5,586,550; 5,758,637, 6,014,970, and 6,085,740.

However, it should be appreciated that the present invention is not limited for use only with such devices.

For example, in one or more embodiments, the aerosol generator is the commercially available Aerogen (Aerogen, Inc. Mountain View, CA) aerosol generator which comprises a vibrational element and dome-shaped aperture plate with tapered holes. When the plate vibrates (at several thousand times per second), a micro-pumping action causes liquid to be drawn through the tapered holes, creating a low-velocity aerosol with a precisely defined range of droplet sizes. The Aerogen aerosol generator does not require propellant.

Jet nebulizers involve use of air pressure to break a liquid solution into aerosol droplets. In one or more embodiments, a jet nebulizer (e.g., Aerojet, AeroEclipse, Pari L. C, the Parijet, Whisper Jet, Microneb®, Sidestream®, Acorn II®, Cirrus and Upmist®) generates droplets as a mist by shattering a liquid stream with fast moving air supplied by tubing from an air pump.

In one or more embodiments, an ultrasonic nebulizer that uses a piezoelectric transducer to transform electrical current into mechanical oscillations is used to produce aerosol droplets. Examples of ultrasonic nebulizers include, but are not limited to, the Siemens 345 UltraSonic Nebulizer™ and ones commercially available from, for example, Omron Heathcare, Inc. and DeVilbiss Health Care, Inc. See, e.g., EP 1 066 850.

Vibrating porous plate nebulizers work by using a sonic vacuum produced by a rapidly vibrating porous plate to extrude a solvent droplet through a porous plate. See, e.g., U.S. Patent Nos. 5,758,637; 5,938,117; 6,014,970; 6,085,740; and 6,205,999.

In condensation aerosol generators, the aerosol is formed by pumping drug formulation through a small, electrically heated capillary. Upon exiting the capillary, the formulation is rapidly cooled by ambient air, and a gentle aerosol is produced that is relatively invariant to ambient conditions and the user inhalation rate. See, e.g., U.S. Patent No. 6,701,922 and WO 03/059413.

In one or more embodiments, the condensation aerosol generator comprises one disclosed by Alexza Molecular Delivery Corporation. See, e.g., U.S. Published Application No. 2004/0096402.

It is understood that while the invention has been described above in connection with preferred embodiments, the description and drawings are intended to illustrate and not limit the scope of the invention, which is defined by the appended claims and their equivalents.

## Claims

1. Apparatus for delivery of a medicament to a breathing system, the apparatus comprising:
a nebulizer that is adapted to aerosolize a liquid medicament to be delivered to a patient's respiratory system; and
a connector (700) that operably connects the nebulizer to a circuit of a pressure-assisted breathing system;
wherein the connector (700) comprises:
a gas conduit (705) having an inlet opening and an outlet opening adapted to be attached to the circuit so that the flow of gas in the circuit is conducted therethrough;
an aerosol supply conduit (702) having an inlet opening (703) adapted to receive the nebulizer and an outlet opening (704) that communicates with the gas conduit (705) so that aerosolized medicament is conducted from the nebulizer to the gas flow in the gas conduit; and
a sealing device comprising a spring-loaded hinged door (706) positioned within the gas conduit (705) adjacent to the aerosol supply conduit (702) outlet opening (704), wherein the nebulizer forces the hinged door (706) into an open position when the nebulizer is received in the aerosol supply conduit (702), and wherein the spring (710) on the hinged door forces the door (706) over the aerosol supply conduit outlet opening (704) when the nebulizer is removed from the aerosol supply conduit, the apparatus **characterized in that**;
said gas conduit comprises a notch or recess (708) in an internal wall; and that
when the hinged door is in the open position it is in cooperation with the notch or recess (708) in the internal wall of the gas conduit (705) thereby allowing unimpeded flow of aerosol from the nebulizer into the gas conduit (705).

2. Apparatus according to claim 1 wherein the door (706) comprises a mating structure which co-operates with said notch or recess (708) to removably hold the door in the open position and/or align the door (706).

## Patentansprüche

1. Vorrichtung zur Abgabe eines Arzneimittels an ein Beatmungssy-stem, wobei die Vorrichtung aufweist:
einen Zerstäuber, der zur Vernebelung eines flüssigen Arzneimittels ausgebildet ist, das an das Atmungssystem eines Patienten abgegeben werden soll; und
einen Verbinder (700), der den Zerstäuber mit einem Kreislauf eines druckunterstützten Beatmungssystems funktionell verbindet;
wobei der Verbinder (700) aufweist:
eine Gasleitung (705) mit einer Einlassöffnung und einer Auslassöffnung, die zum Anbringen an den Kreislauf ausgebildet ist, sodass der Gasstrom in dem Kreislauf durch diese geleitet wird; eine Aerosolzufuhrleitung (702), die eine zur Aufnahme des Zerstäubers ausgebildete Einlassöffnung (703) und eine mit der Gasleitung (705) in Verbindung stehende Auslassöffnung (704) aufweist, sodass ein vernebeltes Arzneimittel von dem Zerstäuber zu dem Gasstrom in der Gasleitung geführt wird; und
eine Verschlusseinrichtung, die eine federbelastete Drehklappe (706) aufweist, die innerhalb der Gasleitung (705) benachbart zur Auslassöffnung (704) der Aerosolzufuhrleitung (702) angeordnet ist, wobei der Zerstäuber die Drehklappe (706) in eine geöffnete Stellung überführt, wenn der Zerstäuber in der Aerosolzufuhrleitung (702) aufgenommen ist, und wobei die Feder (710) an der Drehklappe die Klappe (706) über die Auslassöffnung (704) der Aerosolzufuhrleitung drückt, wenn der Zerstäuber aus der Aerosolzufuhrleitung entfernt wird, wobei die Vorrichtung **dadurch gekennzeichnet ist, dass**
die Gasleitung eine Kerbe oder Vertiefung (708) in einer Innenwand aufweist; und dass
die Drehklappe in der geöffneten Stellung mit der Kerbe oder Vertiefung (708) in der Innenwand der Gasleitung (705) zusammenwirkt, wodurch ein ungehinderter Aerosolstrom von dem Zerstäuber in die Gasleitung (705) ermöglicht wird.

2. Vorrichtung nach Anspruch 1, wobei die Klappe (706) eine passende Gestalt aufweist, die mit der Kerbe oder Vertiefung (708) zusammenwirkt, um die Klappe in der geöffneten Stellung lösbar zu halten und/oder die Klappe (706) zu justieren.

## Revendications

1. Appareil servant à apporter un médicament à un système de respiration, l'appareil comprenant :
un nébuliseur qui est adapté pour pulvériser un médicament liquide devant être administré au système respiratoire d'un patient ; et
un connecteur (700) qui relie de manière fonctionnelle le nébuliseur à un circuit d'un système de respiration assistée en pression ;
dans lequel le connecteur (700) comprend :
un conduit de gaz (705) ayant une ouverture d'entrée et une ouverture de sortie adaptées pour être fixées au circuit de sorte que l'écoulement de gaz dans le circuit est acheminé à travers celui-ci ;
un conduit d'alimentation en aérosol (702) ayant une ouverture d'entrée (703) adaptée pour recevoir le nébuliseur et une ouverture de sortie (704) qui communique avec le conduit de gaz (705) de sorte qu'un médicament pulvérisé soit acheminé du nébuliseur jusqu'à l'écoulement de gaz dans le conduit de gaz ; et
un dispositif d'étanchéité comprenant une porte articulée à ressort (706) positionnée à l'intérieur du conduit de gaz (705) adjacente à l'ouverture de sortie (704) de conduit d'alimentation en aérosol (702), où le nébuliseur pousse la porte articulée (706) dans une position ouverte lorsque le nébuliseur est reçu dans le conduit d'alimentation en aérosol (702), et où le ressort (710) sur la porte articulée pousse la porte (706) au-dessus de l'ouverture de sortie (704) de conduit d'alimentation en aérosol lorsque le nébuliseur est retiré du conduit d'alimentation en aérosol, l'appareil étant **caractérisé en ce que** ;
ledit conduit de gaz comprend une encoche ou un évidement (708) dans une paroi interne ; et **en ce que**
lorsque la porte articulée est dans la position ouverte, elle est en coopération avec l'encoche ou l'évidement (708) dans la paroi interne du conduit de gaz (705), permettant ainsi un écoulement sans entrave de l'aérosol du nébuliseur vers le conduit de gaz (705).

2. Appareil selon la revendication 1, dans lequel la porte (706) comprend une structure correspondante qui coopère avec ladite encoche ou ledit évidement (708) pour maintenir de manière amovible la porte en position d'ouverture et/ou aligner la porte (706).
